# EUROPEAN PATENT SPECIFICATION

(11) **EP 0 836 410 B1**
(45) Date of publication and mention of the grant of the patent: **05.11.2003**
(21) Application number: 97916212.0
(22) Date of filing: 06.03.1997
(51) Int. Cl.: A61B 5/0215

(54) **PRESSURE SENSING GUIDE WIRE**
DRUCKMESS-KATHETER
FIL-GUIDE DE MESURE DE PRESSION

(30) Priority: 07.03.1996 US 614774; 06.09.1996 US 707829
(43) Date of publication of application: 22.04.1998
(73) Proprietor: Scimed Life Systems, Inc., Maple Grove, Minnesota 55311-1566 (US)
(72) Inventor: LAFONTAINE, Daniel, M., Plymouth, MN 55441 (US); TALPADE, Dnyanesh, Plymouth, MN 55441 (US); HASTINGS, Roger, N., Maple Grove, MN 55369 (US); PRATHER, Richard, R., Rogers, MN 55374 (US)
(74) Representative: Dreiss, Fuhlendorf, Steimle & Becker
(86) International application number: PCT/US97/04814
(87) International publication number: WO 97/032518

(56) References cited:
- WO-A-89/10089
- WO-A-95/06433
- WO-A-96/32056
- GB-A- 2 172 203
- US-A- 5 108 369

## Description

The present invention relates to a pressure sensing device having a total compliance, the device comprising an elongate body having a length of about 1-4 meters, a pressure communicating lumen extending therethrough along the whole length of the elongate body,
a proximal end, and a transducer attached to the proximal end of the elongate body and in fluid communication with the lumen.

Such a pressure sending device is known from the prior art section of WO 89 10089 A.

Pressure measurements made before, after, or during a therapeutic or diagnostic procedure can be important methods of analyzing any body conduit. In blood vessels, pressure measurement may be used to continuously monitor a patient's condition, to determine the patency of a specific artery or vessel, to assess the severity of a lesion or stenosis, or to asses the results of a therapeutic procedure such as angioplasty, atherectomy, or stenting. Pressure measurements may be of two types, phasic or flat line where the flat line pressure is the average of pressure changes over time.

One known device measures the pressure as a function of the deflection of a diaphragm located at the distal end of the catheter. While providing the ability to measure both phasic and flat line pressure, positioning the sensing part of the sensing device at the distal end of the catheter requires the sensing device to be made extremely small. Otherwise, the sensing device will impede blood flow and effect the pressure reading. In addition, catheters with the sensing device at the distal end do not allow for reuse of the expensive sensing device.

Another known device, called a fluid filled catheter-manometer, connects a sensing device to the proximal end of the catheter. A catheter-manometer uses a fluid column that can communicate pressure changes at the distal end of the device to a transducer located at the proximal end of the device. A catheter-manometer has the advantage of having a reusable sensor and is therefore less expensive. Unfortunately, catheter-manometers have heretofore not been accurate at physiologic frequencies or able to provide a phasic pressure signal. The required bandwidth, or flat frequency response, for accurate physiologic pressure measurement in the heart about 30 hertz. Large catheter-manometers [8 Fr] 10,4 mm are typically only 20 hertz and small catheter-manometers, like guidewire systems [.014"] 0,356 mm, may be less than 1 hertz. Further the bandwidth of catheter tip pressure devices may be several kilohertz. Clearly, prior art catheter-manometer systems have not had the required bandwidth or frequency response for accurate physiologic or phasic pressure measurement.

There are several factors causing prior art catheter-manometer systems to not have the required bandwidth or frequency response for accurate physiologic pressure measurement. One factor is that a relatively large amount of fluid mass must be moved with a relatively small amount of pressure. The movement of the mass M is described by Newton's second law of motion: F=Ma, where the force F is a combination of a displacing force and restoring force. The displacing force is the pressure input and the restoring force is the natural rebound response of the system. Therefore, the density of the fluid becomes a contributing factor to the frequency response.

Another factor is resistance R. The resistance to flow in a catheter can be described by Poiseuille's equation: R=8ηL/πr⁴, where r is the internal radius η is the viscosity of the fluid and L the length of the catheter. In a fluid-filled manometer system the fluid is typically not flowing, but rather is oscillating, as originally described by Lambossy (1952). Thus, in a fluid filled manometer, the damping (resistance) varies as the square-root of the length and inversely as the cube of the radius.

Yet another factor is the elasticity or compliance C of the system. The catheter combined with the pressure transducer form an elastic volume in which the response is described by: *C* = $\frac{\text{Δ} \text{P}}{\text{Δ} \text{C}}$, wherein ΔV designates a change of volume induced by a pressure change ΔP. Thus the natural frequency response of the fluid filled catheter manometer will be determined by stiffness or compliance of all components in the system. Prior art systems are composed of the catheter, a manifold, tubing, fittings, and a transducer. Catheters are made of combinations of metals and plastics which may, depending on construction, have compliance which absorbs some of the pressure change. Manifolds are made of hard plastics which typically do not elastically deform. However, the tubing used is elastomeric, as are the gaskets used with the fittings. These elastomers are very flexible and will dampen pressure change in the fluid passing through them as described above.

Finally, the responsiveness of the pressure transducer within the sensing device is also a factor. As previously described, all components in the system contribute to the compliance C, and thus the frequency response. In order to have adequate sensitivity in the physiologic pressure range (about [0-5 Psi] 0 - 34500 Pa), blood pressure transducers have traditionally been designed for full scale signal output (maximum pressure rating) at applied pressures of [300 mmHg] 40000 Pa or [5 Psi] 34500 Pa. The very flexible diaphragms used to create adequate low pressure sensitivity increases the compliance of the system, and thus, lowers the frequency response. All of these factors have thus far contributed to making devices of this type less accurate in reproducing the exact physiologic signals and produce pressure readings which are, in some devices, only an average of a patient's blood pressure over time.

The phasic pressure is a wave form. While the average pressure is of interest, physicians can readily identify a true physiologic wave form, with frequency content from DC up to about 30 Hz, and compare it to expected norms, thereby making phasic pressure measurement a highly valuable diagnostic tool.

Percutaneous coronary angioplasty is a specific procedure in which pressure measurement may be a valuable tool for lesion assessment and therapy assessment. The catheter which is used to measure pressure must be small enough so that the catheter itself does not interfere with measurement In the epicardial coronary arteries, this requires catheters which are a fraction of a millimeter in diameter. It is also preferred to make the pressure measurement from a catheter which is already being used in a procedure, rather than exchanging for a pressure measuring catheter.

Prior art devices disclosed by Hastings, et al. in US Patent 5,450,853, Wise, et al. in US Patent 5,113,868, and Little in US Patent 5,313,957 have integrated micro-sensors into the distal end of a guide wire with an electrical or optical interconnect extending to the proximal end of the wire (approximately 1.8 meters). Since the wire is only [0.014 inches] 0,356 mm in outer diameter, it is very difficult to integrate the sensor and interconnect into the guide wire without altering the mechanical performance of the wire. The wire must torque, push, and steer sufficiently well to navigate the tortuous coronary vasculature. Wires with integrated distal sensors which accomplish this feat are inherently expensive to produce.

Prior art fluid lines which provide a phasic pressure signal are typically under-damped and have a diameter much larger than a guide wire. As an example, Model PXMK099 from the Edwards Critical Care division of Baxter Health Care in Irvine, CA consists of a pressure transducer with a [six inch] 152 mm connecting pressure tube connected to a user supplied fluid filled tube. When the Baxter system is connected to a [0.014 inch] 0,356 mm hollow guide wire, the output signal is totally damped and only a flat line average pressure is displayed. This damping is due to the relatively high compliance of the Baxter system and the relatively large volume of water contained therein. To determine the minimum tube diameter which can transmit a phasic blood pressure signal through the Baxter system, 1.8 m long polyimide tubes of varying diameters ranging from [0.012-0.057 inches] 0,305-1,45 mm were connected via a Touey-Borst style connector to the Baxter system. Experiments on this system found an average system compliance of 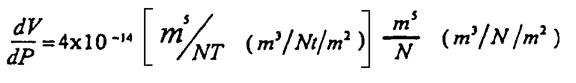 and that the natural frequency was greater than or equal to 30 Hz in lines with diameters greater than [0.053"] 0.0013 m. The lines with diameters less than [0.020 inches] 0,508 mm were over-damped and the lines with diameters larger than [0.020 inches] 0,508 mm were under-damped. Clearly prior art fluid lines which provide adequate frequency response are much larger than guide wires and still are not critically damped.

Another prior art device is disclosed by Tremulis in US Patent 4,953,553. Tremulis discloses a small diameter fluid filled line which can be used as a guide wire. However, blood pressure signals from this device may be extremely damped, giving only an average pressure value.

The above mentioned Document WO 89 10089 A refers to measurements of a pressure differential between two spaced locations in a fluid filled biological vessel. This document mentions fluid filled catheters as the most common type of device for sensing a physiological pressure, the fluid filled catheters extending from the location where the pressure is to be determined to an external pressure transducer outside the patient for recording pressure changes in the fluid filled volumen. Further, this document mentions several problems associated with fluid filled line based pressure measurements. According to this document, an analysis of the rate of pressure change is, generally, not possible with a fluid-filled pressure tracing because of the damping induced by the length and the small diameter of the lumen. A radius of 0.5 mm is given as a minimum diameter of further size reductions. To avoid such damping induced problems, a pressure transducer is arranged at the distal side of an elongated catheter body 12. The pressure transducer is mounted at the proximal side of a dilation mechanism, which includes an elongated dilation balloon having a length of approximately 3 cm.

Lambossy, "Manomètres destinés à l'observation des variations de la pression sanguine", Helv. Physiol. Acta 10, 138 - 160 (1952), discloses that the viscosity of the fluid in the lumen of a medical pressure sensing device of the fluid line type plays an important role because the viscosity causes damping. This document links, inter alia, geometrical dimensions of the fluid line like radius R and length L to characteristics of the fluid like viscosity η and density ρ to a variable E that characterizes the Membrane's dimensions and elasticity. As an example for fluid line dimensions, a length of 6 cm is given.

In view of the above prior art, it is the objective of the present invention to provide a medical pressure sensing device with the reduced cost of a fluid line and a small enough diameter to be used as a guide wire or to be used in small vessels while at the same time being sufficiently responsive to provide a phasic pressure signal.

This objective is achieved by a pressure sensing device as mentioned at the outset wherein the pressure communicating lumen has an inner diameter of less than about 0.0013 meters; and the total compliance of the pressure sensing device is less than about 4∗10⁻¹⁴*m*⁵*N*⁻¹.

The present invention overcomes the deficiencies of the prior art by providing a medical pressure sensing device which may be used as a guide wire, is inexpensive, and responsive enough to measure a phasic pressure signal. A first embodiment of the invention has a tube with an interior diameter of less than 0.0013 m. A pressure transducer is connected to the tube and is in fluid communication with the interior of the tube. Fluid pressure changes at the distal end of the tube are communicated to the pressure transducer at the proximal end of the tube. The system compliance is sufficiently low to measure a phasic signal. In particular the system compliance$\text{C} \text{<} {\left(\frac{\text{D}}{\text{4ƒ} {\text{}}_{\text{0}}}\right)}^{\text{2}} \text{x} \frac{\text{1}}{\text{πρ}}$ and$\text{C} \text{<} {\left(\frac{\text{ξ} \text{D} {\text{}}^{\text{3}}}{\text{32η}}\right)}^{\text{2}} \text{x} \frac{\text{πρ}}{\text{L}} \text{,}$ where the interior diameter D is less than 0.0013 meters, L is the length of the tube, ρ is the density of the fluid, η is the viscosity of the fluid, ƒ₀ is the natural frequency of the system, and ξ is the damping coefficient of the system.

Another embodiment of the medical pressure sensing device is a tube which is about 1-4 meter long and has an inner diameter of less than about 0.0013 m. There is less than about 1 [cc] cm³ of fluid within the tube which transfers pressure changes from the distal end of the tube to a proximal pressure proximal pressure transducer. The total compliance of the system is less than about 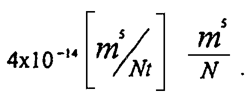 There may also be less than 0.004 [cc] cm³ of air trapped within the tube and the transducer.

The connector described in the previous embodiments may be a separate piece which may be connected to a medical fluid line. Examples of medical fluid lines include guide wires, catheters, needles, etc. The connector may further include a flush port and a stop cock. The total compliance of the connector and the pressure transducer may be less than

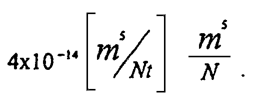

Alternatively, the pressure transducer may be a light source aligned to direct light through the pressure sensing device coupled with a photodetector which is aligned to detect light directed through the fluid line. The light source may be a laser diode and it may be infrared light.

In use, the embodiments of the medical pressure sensing device described above may be inserted into a vessel and advanced to a position where the pressure is desired to be measured. The phasic pressure may then be measured or an average may be computed to provide a pressure measurement. A catheter may then be advanced over the medical pressure sensing device and therapeutic procedures may be conducted. The phasic pressure can be monitored during therapeutic procedures or before and after to determine efficacy of the procedures. The pressure sensing device may also be moved within the vessel to determine the change in pressure measurement across a specific section of the body lumen.

Figure 1a is a side view of a first embodiment of the invention.

Figure 1b is a side view of a membrane covering a hole in the device in Figure 1a.

Figure 1c is a cross section of a second type of membrane covering a hole in the device in Figure 1a.

Figure 2a is a cross section of a another embodiment of the distal end of the device in Figure 1a.

Figure 2b is a cross section of a first variation of the embodiment shown in Figure 2a.

Figure 2c is a cross section of a second variation of the embodiment shown in Figure 2a.

Figure 2d is a cross section of third variation of the embodiment shown in Figure 2a.

Figure 3a is a side view of an embodiment of the connector assembly for use with the invention.

Figure 3b is an end view of the embodiment of Figure 3a.

Figure 3c is a cross section of another embodiment of a connector assembly for use with the invention.

Figure 4a is a cross section of another embodiment of the invention.

Figure 5 depicts a cross section of an alternative embodiment of the invention.

Figure 6 depicts a cross section of an alternative embodiment of the invention.

The following detailed description should be read with reference to the drawings in which like elements in different drawing are numbered identically. The drawings, which are not necessarily to scale, depict selected embodiments and are not intended to limit the scope of the invention.

Examples of constructions, materials, dimensions, and manufacturing processes are provided for selected elements. All other elements employ that which is known to those skilled in the field of the invention. Those skilled in the art will recognize that many of the examples provided have suitable alternatives that may also be used.

A coronary guide wire is an ideal device for measuring pressure. It is the first catheter placed into the artery, and the last to be removed. In addition, since coronary guide wires are typically [0.014 inches] 0,356 mm in diameter, they are also sufficiently smaller than the arteries being treated. Unfortunately, prior art devices described above are too expensive or do not provide adequate signal fidelity. For this reason it is desirable to have a high fidelity fluid line connecting the distal and proximal ends of a guide wire, with a pressure sensor located outside the body where there is ample space to determine pressures at lower cost.

Fluid filled lines have been thoroughly studied and modeled in the past. A fluid line and pressure transducer system are modeled as a simple harmonic oscillator with the mass of fluid in the line, fluid resistance provided by the viscosity of the fluid assuming laminar flow, and restoring force provided by the system compliance. The frequency response of the pressure transducer is then given as a fraction of its dc value by the expression$\frac{\text{A}}{{\text{A}}_{\text{0}}} \text{=} \frac{\text{1}}{\sqrt{{\text{[(ƒ/ƒ}}_{\text{0}} {\text{)}}^{\text{2}} {\text{- 1]}}^{\text{2}} {\text{+ 4ξ(ƒ/ƒ}}_{\text{0}} {\text{)}}^{\text{2}}}} \text{.}$ The natural frequency ƒ₀ can be expressed by the equation${\text{ƒ}}_{\text{0}} \text{=} \frac{\text{D}}{\text{4}} \sqrt{\frac{\text{1}}{\text{π} \text{C} \text{ρ}}} \text{.}$ The total system compliance C equals the sum of the compliance of each of the components in the system, 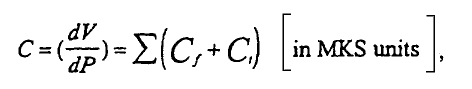 where *C*_{ƒ} equals the compliance of the fluid, any materials that come in contact with the fluid, and any air in the system and *C*_{*t*} equals the compliance of the transducer and any connecting apparatus. The system damping coefficient may be expressed by the equation$\text{ξ =} \frac{\text{32η}}{{\text{D}}^{\text{3}}} \sqrt{\frac{\text{LC}}{\text{πρ}}} \text{.}$

| Symbol | Variable | Units |
|---|---|---|
| L | tube length | meters |
| ξ | system damping coefficient | [NA] |
| ƒ₀ | natural frequency | [HZ] Hz |
| ρ | density of the liquid | kg per cubic meter |
| *A*₀ | amplitude when *f* = 0. | [MKS] |
| A | signal amplitude | [MKS] |
| ƒ | frequency | [HZ] Hz |
| C | system compliance | meters⁵ per Newton [(*m*³/*Nt*/*m*²)] (m³/N/m²) |
| η | fluid viscosity | [*NtS*/*m*²] $\frac{{\text{N}}_{\text{s}}}{{\text{m}}^{\text{2}}}$ |

Commercially available fluid lines which are used to measure patient arterial or venous blood pressure during surgery typically have diameters of a few millimeters or larger and a natural frequency on the order of 30 Hz. Since the natural frequency is proportional to diameter and inversely proportional to the square root of compliance, a guide wire fluid line with a diameter equal to a fraction of a millimeter must have a dramatically reduced system compliance to maintain a 30 Hz or larger bandwidth. Similarly, the damping coefficient depends on the inverse cube of the fluid column diameter with larger diameter conventional lines under damped (ξ < 1). To prevent over damping in small guide wire fluid lines, the compliance of the system must also be dramatically reduced.

Experimental work has confirmed the theoretical work which suggests that the compliance of a hollow [0.014 inch] 0,356 mm guide wire and a proximal pressure transducing apparatus are crucial to the frequency response of the phasic pressure wave. Any soft plastic or rubber materials present, even in small quantities anywhere in the system, can dampen the signal. Wherever possible, minimally compliant materials may then be used. Small amounts of air trapped in the system can also dampen the signal. To achieve adequate bandwidth and a damping coefficient less than unity, the amount of entrapped air should be less than 0.007 mm³. In addition, typical commercially available pressure transducers contain compliant adhesives and gels, which also dampen the signal. If necessary custom pressure transducers may be manufactured. Finally, it has been determined that the total fluid volume of the system should be minimized (less than about [0.1 cc] 0,1 cm³ to transmit good phasic signals. This is because water in a fluid line and connector has a very small but not negligible compliance which is proportional to the total volume of water in the system.

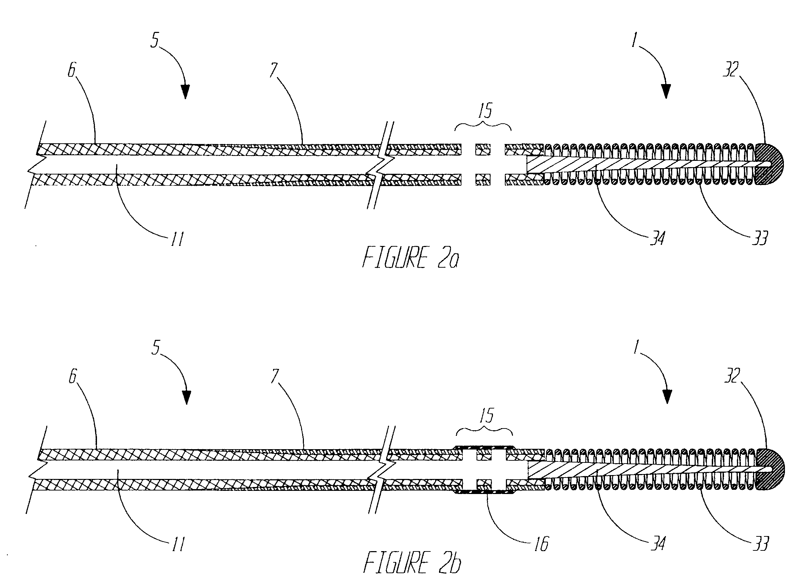

Refer now to Figures 1a-2d, a first embodiment of the invention depicts a guide wire having an elongate body 5 with proximal and distal ends and at least one lumen 11 therethrough. Each lumen 11 has a proximal opening corresponding to the proximal end of elongate body 5 and a distal opening corresponding to the distal end of elongate body 5. At the distal end of elongate body 5 an atraumatic tip 1 is bonded to the distal opening of the lumen.

In Figures 2a-2d an example of an atraumatic tip 1 is shown where the atraumatic tip 1 is a spring tip. Spring tips are well known in the guide wire art and the following description discloses some but not all of the possible materials and methods of construction. A tapered shaft 34 is bonded at its proximal end into the distal opening of lumen 11. Shaft 34 may be welded, adhesively attached, or bonded to elongate body 5 in any way such that a fluid tight and pressure tight seal is formed. Shaft 34 is brazed to a coil 33 at its distal end to form a bead 32. Coil 33 may be made of a radio-opaque material such as, platinum-iridium alloy or other suitable medical grade radio-opaque alloy, to make the spring tip more visible under fluoroscopy. Coil 33 has a diameter of about [.010-.020"] (0,254 - 0,508) mm and encircles shaft 34 along its entire length. Coil 33 is further attached to shaft 34 by welding the proximal portion of coil 33 to the distal end of elongate body 5. Those skilled in the art will recognize that other atraumatic tips 1, such as a polymer covered tip, could be used with the present invention.

Elongate body 5 has holes 15 located near the distal end of elongate body 5. Holes 15 pierce elongate body 5 and make a complete fluid path from the exterior of the distal end of elongate body 5 via lumen 11 to the proximal end of elongate body 5. Holes 15 may be circular, oval, or slot-like and, in the preferred embodiment, each elongate body 5 has at least two and preferably 4 holes 15 in communicating with each lumen 11 therein.

In order to effectively communicate pressure from holes 15 along lumen 11 to the proximal end of elongate body 5, the entire fluid pathway must be so rigid as to be non-compliant, ie. the flexing of materials in contact with the fluid will not absorb any pressure changes. In addition, lumen 11 must be filled with a non-compliant fluid. Suitable fluids include water, heparin, ringers, fluids drugs, saline, silicone oil, gel, silicone gel, alcohol, or perfluorocarbon. These fluids can be pre-filled when the device is manufactured or injected into the device just prior to use. The material that surrounds lumen 11 must also be non-compliant. Suitable materials include Nitinol™ , stainless steel, polycarbonate, or other non-compliant medical grade alloys, polymers, or composites. The resulting structure insures that a displacing force on the exterior of the distal end of elongate body 5 will cause a force equal to the displacing force to be communicated to the proximal end of elongate body 5 via the fluid in lumen 11.

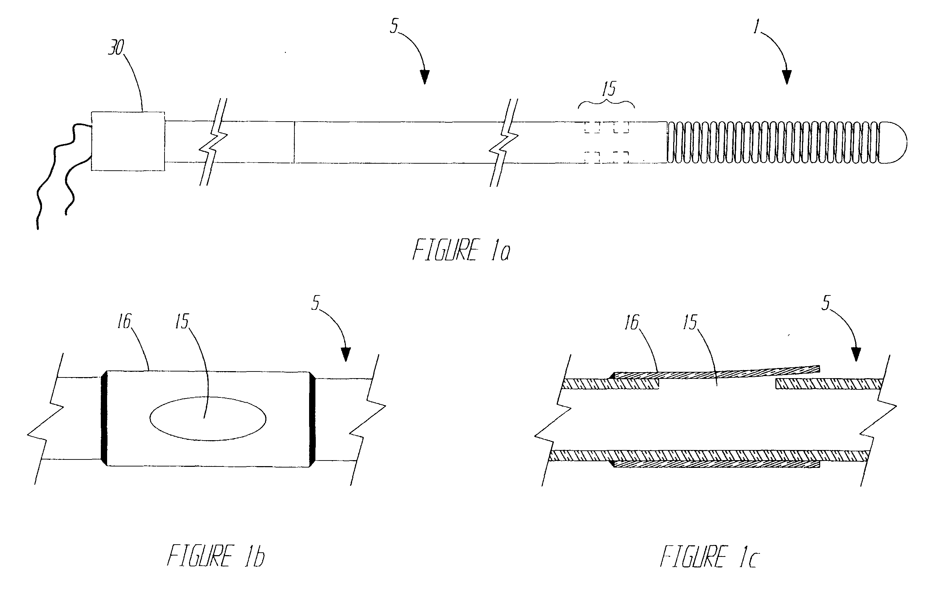

It is also preferable to have each hole 15 act as a one way fluid valve and a two way pressure valve, as shown in Figure 1b and 1c. That is, each hole 15 may allow fluid, including fluid air, to pass only from the interior of elongate body 5 to the exterior while also allowing pressure to be communicated across the valve. This is preferably accomplished by surrounding the hole 15 with a PTFE membrane sleeve 16 as shown in Figure 1b. One example of a material that may be used as the membrane sleeve 16 is Gortex™ with a maximum pore size of [4 microns] 4 µm. Gortex™ is constructed such that its pore structure allows fluid to pass through the membrane in only one direction. As a membrane though, a sleeve made of this material still communicates pressure in either direction. A sleeve of this or other suitable material may be placed over each hole 15 and bonded at each end of the sleeve. Further, a single sleeve may be placed over several holes 15 and bonded on each side of each hole 15.

Alternatively, a membrane sleeve 16 may made be of an elastomer like urethane, a glass fiber composite, or an acrylic copolymer. Use of any of these materials would allow pressure to be communicated across the sleeve 16 but would not allow fluid to pass through the sleeve 16. This type of membrane sleeve 16 may be bonded to the elongate body 5 on only one side, preferably the proximal side, of hole 15 as shown in Figure 1c. In this manner of construction, a positive pressure differential between the interior of elongate body 5 and the exterior of elongate body 5 would force any air and or other fluid out of the interior of elongate body 5 through the unbonded end of the membrane sleeve 16. When the pressure on the exterior of elongate body 5 is equal to or greater than that of the interior, the sleeve 16 would seal the hole 15 to any fluid flow. As an elastomer, sleeve 16 would continue to flex toward the lower pressure side of hole 15 and as such would allow pressure to pass through hole 15 without the transfer of fluid.

Referring back again to Figure 1a, a high pressure transducer 30, preferably with a full scale pressure capability of 10-1,000 times normal blood pressure is sealingly connected and in fluid communication with lumen 11 in the proximal end of elongate body 5. Use of a high pressure transducer which has a very high signal to noise ratio allows physiologic pressure variations to be sensed with only a tiny displacement of the fluid column. Thus relatively rapid pressure variations are communicated accurately through the very small displacement of a small mass of fluid within a non-compliant system. An example of a high pressure transducer 30 may be a silicon piezoresistive pressure transducer, an example of which is the Lucas Nova™ sensor, NPC-102. Pressure transducer 30 preferably has a [1 mmHg] 133 Pa sensitivity and a full scale pressure capability which, as previously described, is 10-1,000 times greater than the range of interest. Where, preferably the use of a [500 psi] 3,45 MPa full scale transducer with the invention has been found to provide good results for measuring blood pressure (approximately [5 psi] 34,5 kPa).

In an alternative embodiment, a dual transducer system may be used to compensate for inaccuracies introduced by variations in the damping coefficient due to mechanical interface variations like air introduced into the system and mechanical tightness. A dual transducer system works by using two identical transducers 30 to create a reference pressure in one transducer 30 and a sensed pressure in the other transducer 30. The reference signal is then filtered to contain a DC or static pressure signal which is subtracted from the dynamic pressure signal. The difference is the offset connect pressure.

Figure 2a shows one approach to the construction of the distal end of the embodiment of Figure 1a. In Figure 2a, elongate body 5 is composed of a core member 6, having a lumen 11 therethrough and an traumatic tip I as previously described. Core member 6 may be made of Nitinol™ or any other suitable medical grade alloy and is preferably made of stainless steel. Core member 6 may be either standard guide wire length, about 150-175 cm, or standard exchangeable guide wire length, about 300 cm. At its proximal end core member 6 has an outer diameter of [.014"] 0,356 mm and an inner diameter of [.008"] 0,203 mm. At the distal end of core member 6 the outer diameter of core member 6 tapers to [.011"] 0,279 mm. A suitable medical grade polymer 7 covers the tapered section of core member 6 making the elongate body have a uniform diameter along its entire length, e.g. [.003"] 0,076 mm of polymer 7 surrounds the distal end of core member 6. Through the distal section of core member 6 are holes 15 which allow fluid communication between the interior and exterior of core member 6. As previously described, holes 15 may be covered by a membrane sleeve 16 as shown in Figure 2b.

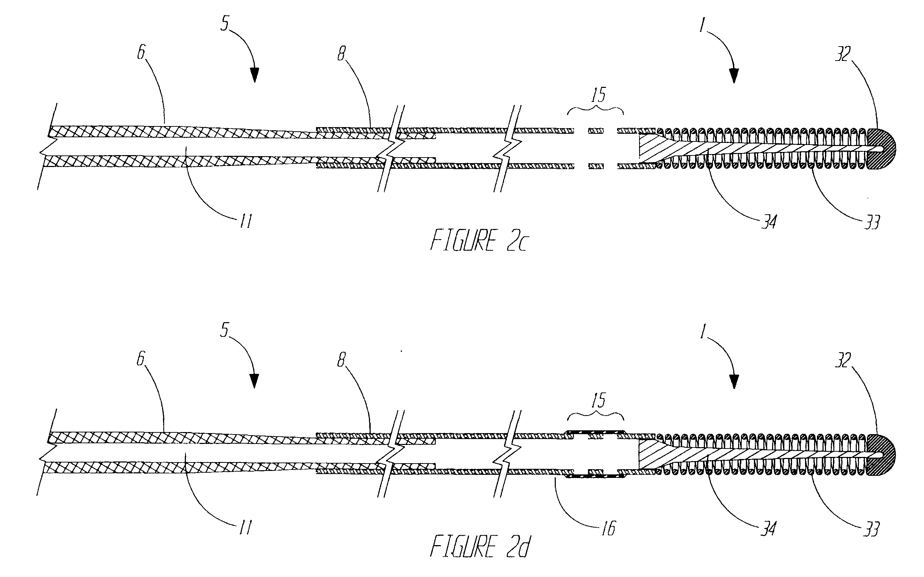

Figures 2c illustrates a second approach to the construction of the distal end of the embodiment of Figure 1a. In Figure 2c elongate body 5 is composed of a core member 6 and hypotube 8. Elongate body 5 has a lumen 11 therethrough and an atraumatic tip 1 as previously described. Core member 6 may be made of stainless steel or any other suitable medical grade alloy and is preferably made of Nitinol™. Elongate body 5 may be either standard guide wire length, about 150-175 cm, or standard exchangeable guide wire length, about 300 cm. The distal end of core member 6 tapers to an outer diameter of [.0105"] 0,267 mm and is suitable for insertion into hypotube 8. The distal end of core member 6 fits within hypotube 8 which has an outer diameter of [.014"] 0,356 mm and an inner diameter of [.0105"] 0,267. Core member 6 may be bonded or press fit together with hypotube 8 and may be made of any medical grade metal but is preferably Nitinol™. Through the distal section of hypotube 8 are holes 15 which allow fluid communication between the interior and exterior of hypotube 8. As previously described, holes 15 may be covered by a membrane sleeve 16 as shown in Figure 2d.

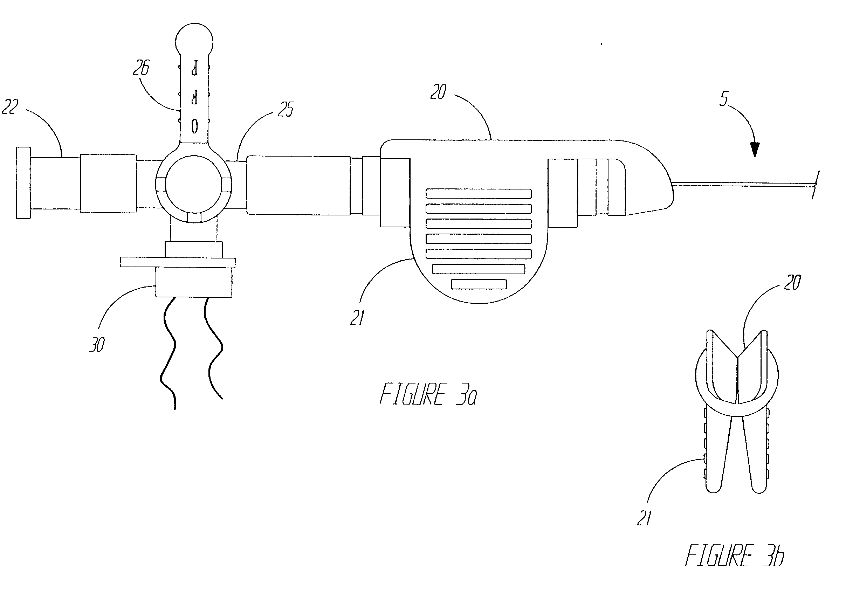

Figures 3a and 3b depict a method of connecting the proximal end of elongate body 5 to a pressure transducer 30. Gripper 20 is a side loading guide wire gripping device as disclosed in US Patent number 4,829,999, herein incorporated by reference. Gripper 20 is molded onto stopcock 25 at its proximal end. Stopcock 25 has a fluid port 22 adapted to receive a syringe and a valve 26 that directs fluid flow. Valve 26 may be aligned to make a fluid path between port 22 and gripper 20, between the gripper 20 and the pressure transducer 30, or to a closed position. Stopcock 25 can be made of any medical grade, non-compressible material and is preferably made of polycarbonate. Figure 3b is an end view of gripper 20.

In Figure 3c a gripper 20 like that of the previous embodiment is molded onto a connector 27. Connector 27 has three ports. The first and second ports are attached to pressure transducers 30. The third port is attached to gripper 20. Within the third port is a neoprene o-ring 28 that provides a fluid tight seal capable of holding a pressurized liquid or gel within connector 27. Examples of these liquids or gels include water, saline, silicone oil, silicone gel, alcohol, or perfluorocarbon. When elongate body 5 is inserted into connector 27 it passes through the o-ring 28 and creates a fluid communication path between the pressure transducers 30 and lumen 11 of elongate body 5.

In use either of the connector assemblies shown in Figures 3a and 3c could be used with either of the tip configurations shown in Figures 2a-2d. However, the actual method of use for each configuration will vary depending on the choice of connector assembly. Use of the connector assembly of Figure 3a and 3b requires the compression of finger pads 21, thereby opening a channel in gripper 20. The core member 6 may then be laid in the channel and then slid into stopcock 25. Finger pads 21 may then be released, thereby securing the hypotube within stopcock 25. The elongate body 5 may then be positively prepped by orienting the valve 26 such that there is a fluid path between the lumen 11 and port 22. A syringe may be fitted into port 22 and fluid injected until all air is forced through holes 15. Valve 26 may then be oriented such that there is a fluid path between holes 15 and the pressure transducer 30. This embodiment would then be used like a standard guide wire.

Use of the connector assembly of Figure 3c requires that the elongate body 5 be positively prepped by injecting a fluid into the proximal end of lumen 11 until all air is expelled from holes 15. The guide wire may then be used like any other guide wire. When the guide wire is located in a position where it is desired to measure pressure, the pre-filled connector assembly of Figure 3c is attached to the proximal end of the core member 6. Compression of finger pads 21 opens a channel in gripper 20. The core member 6 may then be laid in the channel slid through o-ring 28 and into connector 27. Finger pads 21 may then be released, thereby securing the hypotube within connector 27. When transducers 30 are connected to circuitry as describe for a dual sensor system, a pressure measurement can be taken.

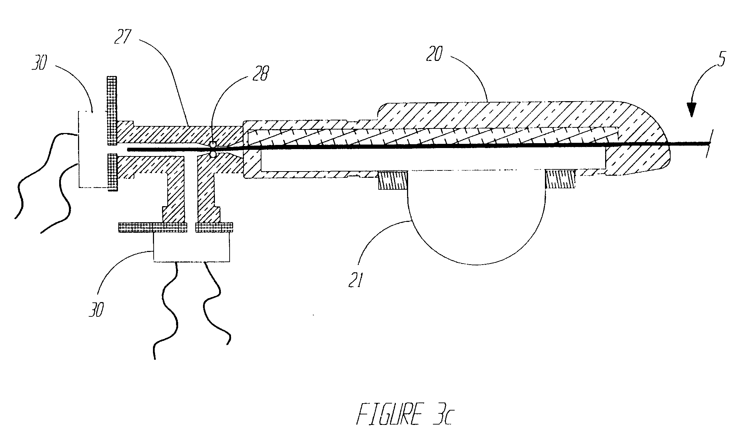

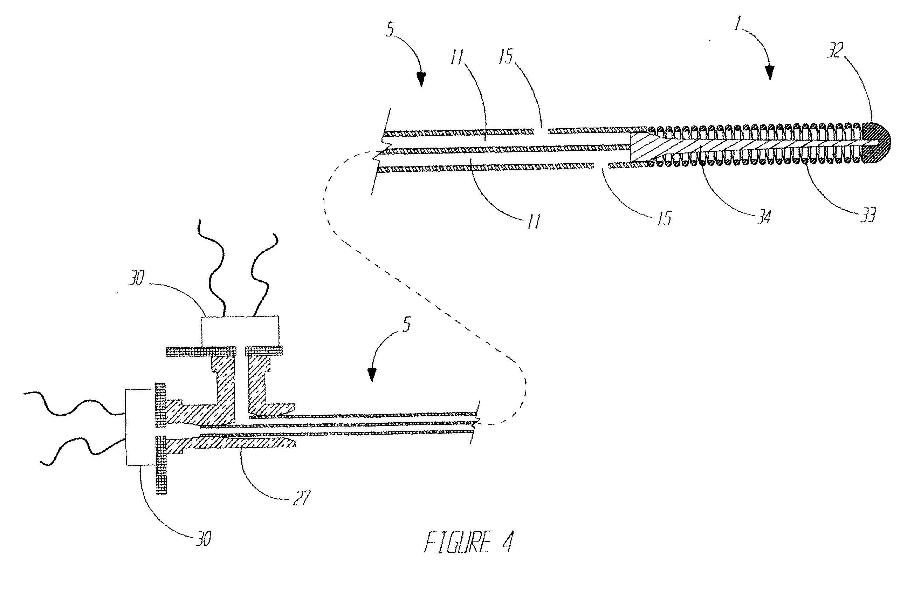

Figure 4 shows an embodiment of the invention with more than one lumen. This embodiment also may have an atraumatic tip 1, as previously described, attached to the distal end of an elongate body 5. Elongate body 5 may be 150-300 cm in length. Elongate body 5 is shown in Figure 4 as having two lumens 11 but may have more than two lumens. Each lumen 11 is in fluid communication with a separate set of holes 15, as previously described. Each set of holes 15 is longitudinally displaced relative to the other sets of holes 15. Longitudinal displacement of each set of holes 15 allows pressure to be measured in multiple locations within the vasculature simultaneously. Each set of holes 15 is in fluid communication, via lumen 11, with a transducer 30, as previously described. Each lumen 11 is formed by a surrounding non-compliant material like stainless steel, polyimide, a polyimide composite, Nitinol ™, or other suitable medical grade metal or polymer. At the proximal end of elongate body 5 there is a connector 27 which is removeably attached to elongate body 5. Alternatively, the embodiment of Figure 4 may also include membrane sleeves 16 covering holes 15 (not shown).

In use, the embodiment of Figure 4 would be prepped be flushing a non-compliant liquid or gel through lumens 11 until all air has been expelled from through holes 15. The device can then be advanced through a patient's vasculature to a point where it is desired to measure pressure. As an example, the device could be advance until the distal set of holes 15 is on the distal side of a vascular lesion. This would leave the proximal set of holes 15 on the proximal side of a lesion. Connector 27 may then be fit onto the proximal end of the device such that each lumen 11 is in fluid communication with a pressure transducer 30. In the example, pressure could then be measured on each side of the lesion. This information will give the users of the device a pressure gradient across the lesion and another tool in determining the extent of the occlusion.

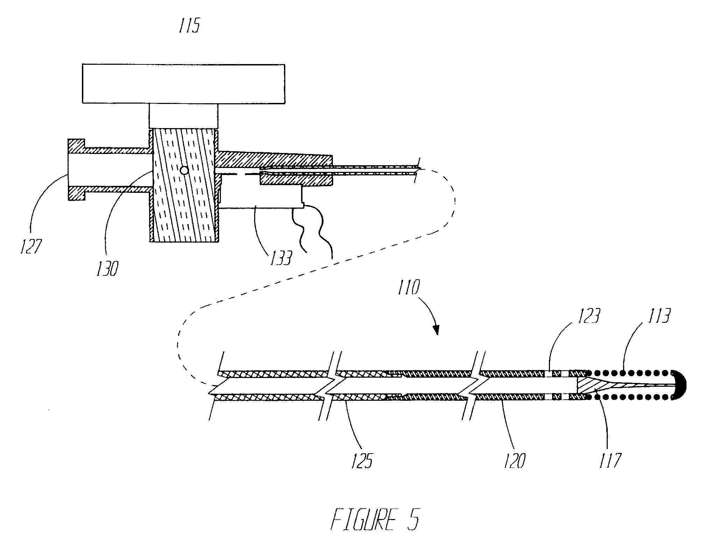

Refer now to Figure 5 which depicts an alternative embodiment of the pressure sensing guide wire where body portion 110. A spring tip 113, as is commonly known in the art, is attached to the distal end of body portion 110. Spring tip 113 may have a safety ribbon 117 and may be about 1-4 cm long and is preferably about 3 cm long. Distal tube 120 may be formed of a super elastic material. The proximal end of safety ribbon 117 may be press fit into distal tube 120, soldered to distal tube 120, or preferably distal tube 120 is chilled into its Martensite phase and then ribbon 117 fit into place. When distal tube 120 is allowed to return to ambient temperature a compressive bond is formed.

Distal tube 120 maybe be any medical grade super elastic material and preferably is Nitinol with an Austinite finish temperature of 10° C ± 10 as supplied by Raychem Corp. of California or the Nitinol Device Corp of California. The outside diameter of distal tube 120 may be about [.0136 inches] 0,345 mm, the inside diameter may be about [.0075 inches] 0,191 mm, and the length may be about [12 inches] 305 mm. Near the distal end of distal tube 120 are holes 123. There may be as few as one hole 123 but preferably there are about 6 holes 123 arranged in a helical pattern around distal tube 120 and spaced along an axial length of [.020-.040 inches] 0,508 - 1,016 mm. While no more than one hole 123 is required to provide a phasic pressure signal, several holes 123 ensure that the vessel wall or other material does not plug distal tube 120. Holes 123 may be electron discharge milled into distal tube 120 and electro-etched to remove any burrs. The exterior of the distal end of distal tube 120 may be further electro-etched to increase the flexibility of distal tube 120.

Alternatively, distal tube 120 may be made of a polymer/wire composite (not shown) as disclosed in WO 93/20881 to Pray et al., which is herein incorporated by reference. There may be one or more wires arranged in one of a variety of different patterns such as helix. This alternative distal tube 120 may be more flexible than a Nitinol distal tube 120 while maintaining a minimally compliant fluid path and the performance characteristics of a coronary guide wire.

The distal end of proximal hypotube 125 is bonded to the proximal end of distal tube 120. Proximal hypotube 125 may be press fit into distal tube 120, soldered to distal tube 120, or bonded in the method previously described. The joint between proximal hypotube 125 and distal tube 120 may be a stepped joint. However, to reduce the likelihood of breakage an angled joint *as* shown in Figure 5 is preferred. Proximal hypotube 125 may be made of any medical grade alloy and is preferably made of 304V stainless steel which may be heat treated for resilience and plug drawn for smoothness. The inside diameter of proximal hypotube 125 may be about [.0075 inches] 0,191 mm, the outside diameter about [.0136 inches] 0,345 mm, and the length about [160 inches] 406 cm.

Flushing connector 115 may be bonded to the proximal end of proximal hypotube 125 or flushing connector 115 may be adapted to be releasably connected to any physiological fluid line. Flushing connector 115 may have a flushing port 127 and a stop cock 130. Integrally formed with or bonded to flushing connector 115 is pressure transducer 133. Pressure transducer 133 may be a commercially available solid state pressure transducer such as Model #109 available from Lucas Nova Corporation, in Freemont, CA. Alternatively a custom pressure transducer may be manufactured by modifying the Model #109 from Lucas Nova Corporation which reduces the RTV glue used to bond the sensor to the substrate. The pressure transducer 133 may have electrical leads suitable for connection to standard monitoring systems.

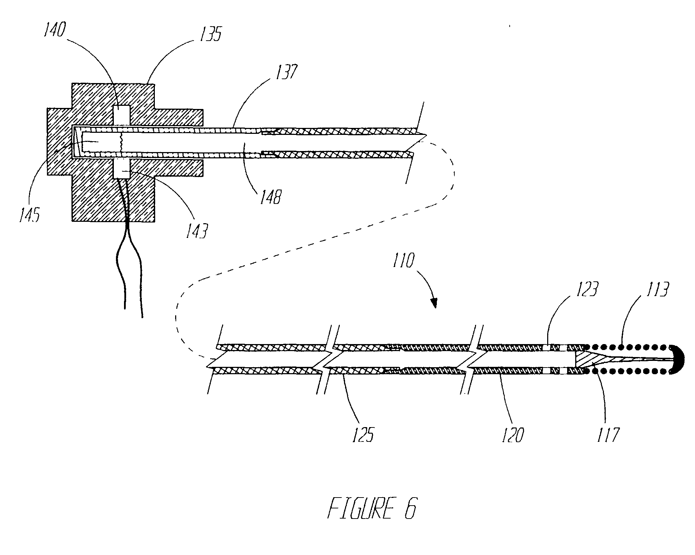

Figure 6 depicts a another embodiment of the invention with body section 110 the same as previously described for the embodiment of Figure 5. An optically clear view tube 137 is bonded to the proximal portion of proximal hypotube 125. View tube 137 may be made of glass, polyimide, a fused silica capillary as sold by Polymicro Technologies of Phoenix, AZ, or any other optically clear minimally compliant material. Optical connector 135 may be bonded or releasably attached to the exterior of the proximal end of proximal hypotube 125 or may be adapted to be releasably connected to any physiological fluid line. Optical connector 135 houses a light source 140 which is aligned to shine through view tube 137. Light source 140 may be a light bulb with a lens or a laser diode but preferably is a light emitting diode. Light source 140 may produce a variety of wavelengths of light and will preferably produce infrared light. Optical connector 135 may also house a photodetector 143 aligned to receive light from light source 140 that has passed through view tube 137.

Within the proximal end of view tube 137 is an air column 145. Air column 145 is trapped by pressure communicating fluid 148. Pressure communicating fluid 148 may be any fluid which is bio-compatible, opaque to light from light source 140, minimally evaporative, and non-corrosive, examples of which may include ferrofluids, cotton seed oil, vegetable oil, saline, and water. The guide wire of this embodiment may be prepped prior to packaging. Specifically an air column 145 needs to be put in place in the proximal end of view tube 137, the pressure communicating fluid 148 loaded into view tube 137, and a temporary seal (not shown) placed around holes 123. The interface between the air column 145 and the pressure communicating fluid 148 must be aligned so as to cause a shadow between light source 140 and photodetector 143. Changes in fluid pressure at the distal end of the guide wire will cause the interface to move and the change in light detected by the photodetector can be interpreted as pressure changes. Preferably an air column which is about [0.01 inches] 0,254 mm long in a tube of [0.008 inches] 2,03 mm inside diameter is desired to give a compliance of about 8x10⁻¹⁷ [*m*⁵/*Nt*] $\frac{{\text{m}}^{\text{5}}}{\text{N}}$ and corresponds to a natural frequency of about 100 Hz and a damping coefficient of near unity. Further, the guide wire may be connected to a view tube 137 which has an outside diameter which is smaller than the inside diameter of the guide wire thereby allowing linear movement of the fluid column for a given change in distal pressure.

In use the embodiments shown in Figures 5 and 6 may be prepped by the manufacturer prior to packaging or by the user just prior to the procedure. When prepped by the user, the common technique of a positive prep may be used. This technique involves flushing fluid from the proximal end the guide wire, out of the distal end of the guide wire and thereby flushing any air from the system. A negative prep may also be used by creating negative pressure at the proximal end of the guide wire, as by a syringe, and drawing any air from the system while filling the guide wire with a fluid. Once prepped, the guide wire may be used in the same way as a conventional guide wire. That is, the wire is inserted into the vasculature and advanced to a desired treatment site. Once at the treatment site the guide wire, unlike common coronary guide wires, may be used to sense phasic pressure. Pressure may be sensed at different locations. For instance pressure can be measured on either side of a lesion. If a therapeutic procedure is desired, another device, like an angioplasty balloon catheter, may be advanced over the wire. Pressure may also be measured at different times such as during, before, or after a procedure.

## Claims

1. A pressure sensing device having a total compliance, the device comprising:
an elongate body (5) having a length of about 1-4 meters;
a pressure communicating lumen (11) extending therethrough along the whole length of the elongate body (5);
a proximal end; and
a transducer (31; 133) attached to the proximal end of the elongate body (5) and in fluid communication with the lumen (11) by means of a pressure communication fluid (148) ;
**characterized by**
the pressure communicating lumen (11) having an inner diameter of less than about 0.0013 meters, and
the total compliance of the pressure sensing device being less than about 4∗10⁻¹⁴*m*⁵*N*⁻¹.

2. The pressure sensing device of claim 1 wherein the total compliance of the device includes the compliance of a volume of fluid (148) in the device, the volume of fluid (148) being less than about one cubic centimeter.

3. The pressure sensing device of claim 1 wherein the total compliance of the device includes the compliance of a volume of entrapped air disposed in the device, the volume of entrapped air being less than about 0.004 cubic centimeters.

4. The pressure sensing device of claim 1, **characterized by** having a connector (27), the connector comprising:
a body portion 110 having an interior and a port suitable for providing fluid communication between the medical pressure sensing instrument and the interior of the body portion (110); the body portion (110) having a compliance; and
a pressure transducer (31; 133) attached to the body portion (110) and in fluid communication with the interior of the body, the pressure transducer (31; 133) having a compliance, wherein the sum of the compliance of the body portion (110) and the compliance of the pressure transducer (31; 133) is less than 4∗10⁻¹⁴*m*⁵*N*⁻¹.

5. The pressure sensing device of claim 4, **characterized by** the connector further comprising:
a flush port(22; 127) in fluid communication with the interior of the body portion (110); and
a stop cock (25, 130) positioned such that fluid communication between the interior of the body portion (110) and the flush port (22; 127) may be blocked.

6. The pressure sensing device of claim 4, **characterized in that** the pressure transducer (31; 133) is a solid state pressure transducer.

7. the pressure sensing device of claim 4, **characterized by** the pressure transducer (31; 133) comprising:
a light source (140) attached to the body portion (110) and aligned to shine through the medical pressure sensing instrument; and
a photodetector (143) attached to the body portion (110) and positioned to detect light shined through the medical sensing instrument.

8. The pressure sensing device of claim 7, **characterized in that** the light source is a light emitting diode.

9. The pressure sensing device of claim 7, **characterized in that** the light source (140) emits infrared light.

10. The pressure sensing device of claim 4, **characterized by** at least one hole (15; 123) near the distal end of the elongate body (5) provide pressure communication between the lumen (11) and a pressure source external to the elongate body (5), the at least one pressure transducer (31; 133) being adapted to have a full scale pressure capability which is 10-16,000 times greater than the range of normal blood pressure.

11. The pressure sensing device of claim 10 **characterized in that** the fluid (148) is a fluid selected from the group consisting of water, saline, silicone oil, perfluorocarbon, gels, silicone gel, and alcohol.

12. The pressure sensing device of claim 11 **characterized in that** at least one valve (26) is attached to the distal end of the elongate body (5) such that the at least one valve (26) covers the at least one hole (15; 123).

13. The pressure sensing device of claim 12, **characterized in that** the valve 26 allows fluid (148) to flow out of the lumen (11) but not into the lumen (11) and allows pressure to be communicated across the valve (26).

14. The pressure sensing device of claim 13, **characterized in that** the valve (26) comprises a PTFE membrane attached to the distal end of the elongate body (5) to cover the hole (15, 123).

15. The pressure sensing device of claim 10, **characterized in that** the connector (27) has a first port adapted to connect to the proximal end of the elongate body (5); a second port adapted to connect to a pressure transducer (31; 133), and at least one additional port.

16. The pressure sensing device of claim 10, **characterized in that** the connector (27) is made of polycarbonate.

17. The pressure sensing device of claim 10, **characterized in that** two pressure transducers (31; 133) are in fluid communication with each at least one lumen (11).

## Patentansprüche

1. Druckerfassungsvorrichtung mit einer Gesamt-Compliance, welche Vorrichtung enthält:
einen länglichen Körper (5) mit einer Länge von etwa 1-4 Metern;
ein Druck übertragendes Lumen (11), das durch diesen über die gesamte Länge des länglichen Körpers (5) verläuft;
ein proximales Ende; und
einen Transducer (31; 133), der an dem proximalen Ende des länglichen Körpers (5) angebracht ist und mit dem Lumen (11) mittels eines Druckübertragungsfluids (148) in Fluidverbindung steht;
**dadurch gekennzeichnet, dass**
das Druck übertragende Lumen (11) einen inneren Durchmesser von weniger als etwa 0,0013 Metern hat, und
die Gesamt-Compliance der Druckerfassungsvorrichtung weniger als etwa 4*10⁻¹⁴*m*⁵*N*⁻¹ beträgt.

2. Druckerfassungsvorrichtung nach Anspruch 1, bei welcher die Gesamt-Compliance der Vorrichtung die Compliance eines Fluidvolumens (148) in der Vorrichtung enthält, welches Fluidvolumen (148) weniger als etwa ein Kubikzentimeter ist.

3. Druckerfassungsvorrichtung nach Anspruch 1, bei welcher die Gesamt-Compliance der Vorrichtung die Compliance eines Volumens von in der Vorrichtung angeordneter, eingeschlossener Luft einschließt, welches Volumen eingeschlossener Luft weniger als etwa 0,004 Kubikzentimeter beträgt.

4. Druckerfassungsvorrichtung nach Anspruch 1, **dadurch gekennzeichnet, dass** sie ein Verbindungsstück (27) hat, welches Verbindungsstück enthält:
einen Körperabschnitt (110), der ein Inneres und eine Öffnung hat, die geeignet sind, um eine Fluidverbindung zwischen dem medizinischen Druckerfassungsinstrument und dem Inneren des Körperabschnitts (110) zu schaffen; welcher Körperabschnitt (110) eine Compliance hat; und
einen Drucktransducer (31; 133), der an dem Körperabschnitt (110) angebracht ist und in Fluidverbindung mit dem Inneren des Körpers steht, welcher Drucktransducer (31; 133) eine Compliance hat, wobei die Summe der Compliance des Körperabschnitts (110) und die Compliance des Drucktransducers (31; 133) weniger als 4*10⁻¹⁴*m*⁵*N*^{*-1*} beträgt.

5. Druckerfassungsvorrichtung nach Anspruch 4, **dadurch gekennzeichnet, dass** das Verbindungsstück ferner enthält:
eine Spülöffnung (22; 127), die in Fluidverbindung mit dem Inneren des Körperabschnitts (110) steht; und
ein Absperrventil (25, 130), das so positioniert ist, dass die Fluidverbindung zwischen dem Inneren des Körperabschnitts (110) und der Spülöffnung (22; 127) gesperrt werden kann.

6. Druckerfassungsvorrichtung nach Anspruch 4, **dadurch gekennzeichnet, dass** der Drucktransducer (31; 133) ein Festkörper-Drucktransducer ist.

7. Druckerfassungsvorrichtung nach Anspruch 4, **dadurch gekennzeichnet, dass** der Drucktransducer (31; 133) enthält:
eine Lichtquelle (140), die an dem Körperabschnitt (110) angebracht ist und so ausgerichtet ist, dass sie durch das medizinische Druckerfassungsinstrument strahlt; und
einen Photodetektor (143), der an dem Körperabschnitt (110) angebracht ist und so positioniert ist, dass er durch das medizinische Erfassungsinstrument gestrahltes Licht erfasst.

8. Druckerfassungsvorrichtung nach Anspruch 7, **dadurch gekennzeichnet, dass** die Lichtquelle eine Licht emittierende Diode ist.

9. Druckerfassungsvorrichtung nach Anspruch 7, **dadurch gekennzeichnet, dass** die Lichtquelle (140) Infrarotlicht emittiert.

10. Druckerfassungsvorrichtung nach Anspruch 4, **gekennzeichnet durch** mindestens ein Loch (15; 123) nahe dem distalen Ende des länglichen Körpers (5), um eine Druckübertragung zwischen dem Lumen (11) und einer Druckquelle außerhalb des länglichen Körpers (5) zu schaffen, wobei der mindestens eine Drucktransducer (31; 133) so ausgelegt ist, dass er ein Druckvermögen im vollen Umfang hat, das 10-16.000 mal größer als der Bereich des normalen Blutdrucks ist.

11. Druckerfassungsvorrichtung nach Anspruch 10, **dadurch gekennzeichnet, dass** das Fluid (148) ein Fluid ist, das ausgewählt ist aus der Gruppe bestehend aus Wasser, Kochsalzlösung, Siliconöl, Perflurkohlenstoff, Gelen, Silicongel und Alkohol.

12. Druckerfassungsvorrichtung nach Anspruch 11, **dadurch gekennzeichnet, dass** mindestens ein Ventil (26) an dem distalen Ende des länglichen Körpers (5) in der Weise angebracht ist, dass das mindestens eine Ventil (46) das mindestens eine Loch (15; 193) abdeckt.

13. Druckerfassungsvorrichtung nach Anspruch 12, **dadurch gekennzeichnet, dass** das Ventil (26) zulässt, dass Fluid (148) aus dem Lumen (11) fließt, jedoch nicht in das Lumen (11), und die Übertragung von Druck über das Ventil (26) erlaubt.

14. Druckerfassungsvorrichtung nach Anspruch 13, **dadurch gekennzeichnet, dass** das Ventil (26) eine PTFE-Membran enthält, die an dem distalen Ende des länglichen Körpers (5) angebracht ist, sodass sie das Loch (15, 123) bedeckt.

15. Druckerfassungsvorrichtung nach Anspruch 10, **dadurch gekennzeichnet, dass** das Verbindungsstück (27) eine erste Öffnung, die zur Verbindung mit dem proximalen Ende des länglichen Körpers (5) ausgelegt ist; eine zweite Öffnung, die zur Verbindung mit einem Drucktransducer (31; 133) ausgelegt ist, und mindestens eine zusätzliche Öffnung hat.

16. Druckerfassungsvorrichtung nach Anspruch 10, **dadurch gekennzeichnet, dass** das Verbindungsstück (27) aus Polycarbonat hergestellt ist.

17. Druckerfassungsvorrichtung nach Anspruch 10, **dadurch gekennzeichnet, dass** zwei Drucktransducer (31; 133) in Fluidverbindung mit mindestens einem Lumen (11) stehen.

## Revendications

1. Dispositif de détection de pression ayant une élasticité totale, le dispositif comprenant :
un corps allongé (5) ayant une longueur d'environ 1-4 mètres ; un lumen qui communique la pression (11) s'étendant à travers celui-ci sur toute la longueur du corps allongé (5) ;
une extrémité proximale ; et
un transducteur (31 ; 133) fixé à l'extrémité proximale du corps allongé (5) et en communication de fluide avec le lumen (11) au moyen d'un fluide de communication de pression (148) ;
**caractérisé en ce qu'**il comprend :
le lumen qui communique la pression (11) ayant un diamètre interne inférieur à environ 0,0013 mètre, et
l'élasticité totale du dispositif de détection de pression étant inférieure à environ 4*10⁻¹⁴ m⁵N⁻¹.

2. Dispositif de détection de pression selon la revendication 1 dans lequel l'élasticité du dispositif comprend l'élasticité d'un volume de fluide (148) dans le dispositif, le volume de fluide (148) étant inférieur à environ un centimètre cube.

3. Dispositif de détection de pression selon la revendication 1 dans lequel l'élasticité totale du dispositif comprend l'élasticité d'un volume d'air piégé disposé dans le dispositif, le volume d'air piégé étant inférieur à environ 0,004 centimètre cube.

4. Dispositif de détection de pression selon la revendication 1, **caractérisé en ce qu'**il comprend un connecteur (27), le connecteur comprenant :
une partie de corps 110 ayant un intérieur et un orifice approprié pour proposer la communication de fluide entre l'instrument médical de détection de pression et l'intérieur de la partie de corps (110) ; la partie de corps (110) présentant une certaine élasticité ; et
un transducteur de pression (31 ; 133) fixé à la partie de corps (110) et en communication de fluide avec l'intérieur du corps, le transducteur de pression (31 ; 133) ayant une certaine élasticité, dans lequel la somme de l'élasticité de la partie de corps (110) et l'élasticité du transducteur de pression (31 ; 133) est inférieure à 4*10⁻¹⁴ m⁵N⁻¹.

5. Dispositif de détection de pression selon la revendication 4, **caractérisé en ce qu'**il comprend le connecteur comprenant en outre :
un orifice de vidange (22 ; 127) en communication de fluide avec l'intérieur de la partie de corps (110) ; et
un robinet d'arrêt (25, 130) positionné de sorte que la communication de fluide entre l'intérieur de la partie de corps (110) et l'orifice de vidange (22 ; 127) peut être bloqué.

6. Dispositif de détection de pression selon la revendication 4, **caractérisé en ce que** le transducteur de pression (31 ; 133) est un transducteur de pression à l'état solide.

7. Dispositif de détection de pression selon la revendication 4, **caractérisé en ce qu'**il comprend le transducteur de pression (31 ; 133) comprenant :
une source lumineuse (140) fixée à la partie de corps (110) et alignée pour briller à travers l'instrument médical de détection de pression ; et
un photodétecteur (143) fixé sur la partie de corps (110) et positionné pour détecter la lumière qui brille à travers l'instrument médical de détection.

8. Dispositif de détection de pression selon la revendication 7, **caractérisé en ce que** la source lumineuse est une diode électroluminescente.

9. Dispositif de détection de pression selon la revendication 7, **caractérisé en ce que** la source lumineuse (140) émet de la lumière infrarouge.

10. Dispositif de détection de pression selon la revendication 4, **caractérisé en ce qu'**il comprend au moins un trou (15 ; 123) situé près de l'extrémité distale du corps allongé (5) qui propose la communication de pression entre le lumen (11) et une source de pression externe au corps allongé (5), le au moins un transducteur de pression (31 ; 133) étant adapté pour avoir une capacité de pression à pleine échelle qui est 10-16.000 fois supérieure à l'intervalle de pression sanguine normal.

11. Dispositif de détection de pression selon la revendication 10 **caractérisé en ce que** le fluide (148) est un fluide choisi dans le groupe comprenant l'eau, la solution saline, l'huile de silicone, l'hydrocarbure perfluoré, les gels, le gel de silicone, et l'alcool.

12. Dispositif de détection de pression selon la revendication 11 **caractérisé en ce qu'**au moins un clapet (26) est fixé à l'extrémité distale du corps allongé (5) de sorte qu'au moins un clapet (26) couvre le au moins un trou (15 ; 123).

13. Dispositif de détection de pression selon la revendication 12, **caractérisé en ce que** le clapet 26 permet au fluide (148) de s'écouler hors du lumen (11) mais pas dans le lumen (11) et permet à la pression d'être communiquée sur le clapet (26).

14. Dispositif de détection de pression selon la revendication 13, **caractérisé en ce que** le clapet (26) comprend une membrane de PTFE fixée sur l'extrémité distale du corps allongé (5) pour couvrir le trou (15, 123).

15. Dispositif de détection de pression selon la revendication 10, **caractérisé en ce que** le connecteur (27) est muni d'un premier orifice adapté pour se raccorder à l'extrémité proximale du corps allongé (5) ; d'un second orifice adapté pour se raccorder à un transducteur de pression (31 ; 133), et d'au moins un orifice supplémentaire.

16. Dispositif de détection de pression selon la revendication 10, **caractérisé en ce que** le connecteur (27) est réalisé en polycarbonate.

17. Dispositif de détection de pression selon la revendication 10, **caractérisé en ce que** les deux transducteurs de pression (31 ; 133) sont en communication de fluide avec chaque au moins un lumen (11).
